# EUROPEAN PATENT SPECIFICATION

(11) **EP 1 101 605 B1**
(45) Date of publication and mention of the grant of the patent: **07.06.2006**
(21) Application number: 00124827.7
(22) Date of filing: 14.11.2000
(51) Int. Cl.: B32B 27/08, A61F 5/445

(54) **Multilayer chlorine-free film with barrier layer of a polyamide blend and ostomy pouches formed therefrom**
Chlorfreie Mehrschichtfolie mit einer Sperrschicht aus einer Polyamidmischung und daraus hergestellte Ostomiebeutel
Feuille stratifiée sans chlore avec une couche barrière en mélange de polyamide et poches d'ostomy à partir de cette feuille

(30) Priority: 22.11.1999 US 444813
(43) Date of publication of application: 23.05.2001
(73) Proprietor: HOLLISTER INCORPORATED, Libertyville, Illinios 60048 (US)
(72) Inventor: Giori, Claudio, Riverwoods, Illinois 60015 (US)
(74) Representative: Grünecker, Kinkeldey, Stockmair & Schwanhäusser Anwaltssozietät

(56) References cited:
- EP-A- 0 622 182
- EP-A- 0 700 777
- US-A- 5 567 489
- US-A- 5 985 390

## Description

Films for ostomy applications should have good odor barrier properties and produce minimal noise when flexed or wrinkled to avoid embarrassment to users. Typically, films currently in use for ostomy applications utilize polyvinylidene chloride (PVDC) or copolymers of vinylidene chloride with a comonomer such as methylacrylate or vinylchloride as the gas barrier layer of a multilayer film. Such multilayer films have good resistance to odor transmission and are also relatively quiet; however, they are also believed to be hazardous to the environment when disposed of by incineration, a common practice in numerous countries. Chlorinated polymers generate hydrochloric acid as a byproduct of incineration and are believed to be a significant contributor to hydrochloric acid release from incinerator flue gases. Furthermore, chlorinated polymers are believed to form toxic dioxin derivatives as byproducts of incineration which are retained in the ashes and may possibly cause solid waste disposal problems.

Unfortunately, films formed of chlorine-free barrier resins tend to be stiffer and noisier than films utilizing conventional PVDC-based resins and do not match the quality of conventional chlorinated films for use in ostomy appliances. Thus, a need exists for a multilayer film which is chlorine-free, can be manufactured by coextrusion from readily available raw materials, is heat sealable, has high softness and low noise when flexed or wrinkled, and is impermeable to fecal odors.

U.S. patent 5,567,489 discloses a multilayer barrier film in which a chlorine-free barrier layer is composed of amorphous nylon, crystalline nylon, copolymers of ethylene and vinyl alcohol, or blends thereof. Although data presented in the patent indicate the multilayer films to be comparable in quietness to some chlorinated films in general commercial use, experience has revealed that such chlorine-free films are nevertheless significantly noisier than the chlorine-containing films commonly employed for the fabrication of ostomy pouches. The general observation is that chlorine-free barrier resins are high modulus, stiff materials that do not lend themselves to the production of low noise ostomy films. This is true of all nylon (polyamide) barrier resins, both crystalline and amorphous. It is true also of other known chlorine-free barrier resins such as hydrolyzed ethylene-vinylacetate copolymers, commonly known as ethylene-vinylalcohol copolymers, and copolymers of acrylonitrile or methacrylonitrile of high nitrile content, commonly known as nitrile resins.

Other references illustrating the current state of the art relating to chlorine-free multilayer films are U.S. patents 5,496,295, 5,643,375, 5,407,713, and 5,895,694. Based on the drawbacks of the prior art the present invention provides the products as defined in claims 1 to 19.

An important aspect of this invention lies in the discovery that the noise properties of a multilayer film in which amorphous nylon (polyamide) is utilized for the odor barrier layer may be significantly reduced, without appreciably affecting the barrier properties, by blending the nylon with an anhydride-modified olefinic polymer or copolymer having a density of 0.89g/cc or lower. The anhydride-modified olefinic polymer or copolymer should be present in the range of 10% to 30%, preferably 15% to 25%, per total weight of the barrier layer.

The multilayer film includes at least one skin layer, preferably two such skin layers, consisting essentially of an ethylene polymer or copolymer, and an adhesive tie layer interposed between each skin layer and the blended amorphous nylon barrier layer. Each adhesive tie layer is primarily composed of an anhydride-modified ethylenic polymer, such as polyethylene or copolymer of ethylene and vinylacetate, containing anhydride groups capable of promoting interfacial adhesion with the polyamide-containing barrier layer.

The result is a heat-sealable multilayer film that is particularly useful for ostomy appliances because of its exceptional odor barrier properties while at the same time being relatively soft (low modulus) and quiet in relation to known chlorine-free films in which the odor barrier layer is formed entirely of nylon, ethylene- vinylalcohol copolymers, or nitrile resins. With regard to the generation of noise upon flexing, the chlorine-free multilayer films of this invention compare favorably with prior art ostomy films having chlorinated barrier layers. A pouch formed of the multilayer film of this invention therefore has properties comparable to those exhibited by high-quality pouches formed of chlorine-containing compositions but without the environmental shortcomings described above.

Other features, advantages and objects of the invention will become apparent from the specification and drawings.

### Drawings

Figure 1 is a schematic cross-sectional view of an embodiment of the multilayer barrier film of this invention.
Figure 2 is an elevational view of an ostomy pouch formed from the multilayer barrier film of Figure 1.

### Detailed Description of Preferred Embodiments

The multilayer chlorine-free film of the present invention may be produced using standard coextrusion techniques involving either casting or blowing. Preferably, the multilayer film has five layers -- a chlorine-free barrier layer sandwiched between two heat-sealable skin layers with tie layers interposed between the skin and barrier layers -- but the advantages of the invention may be at least partially achieved in a three-layer structure having a barrier layer and single skin and tie layers.

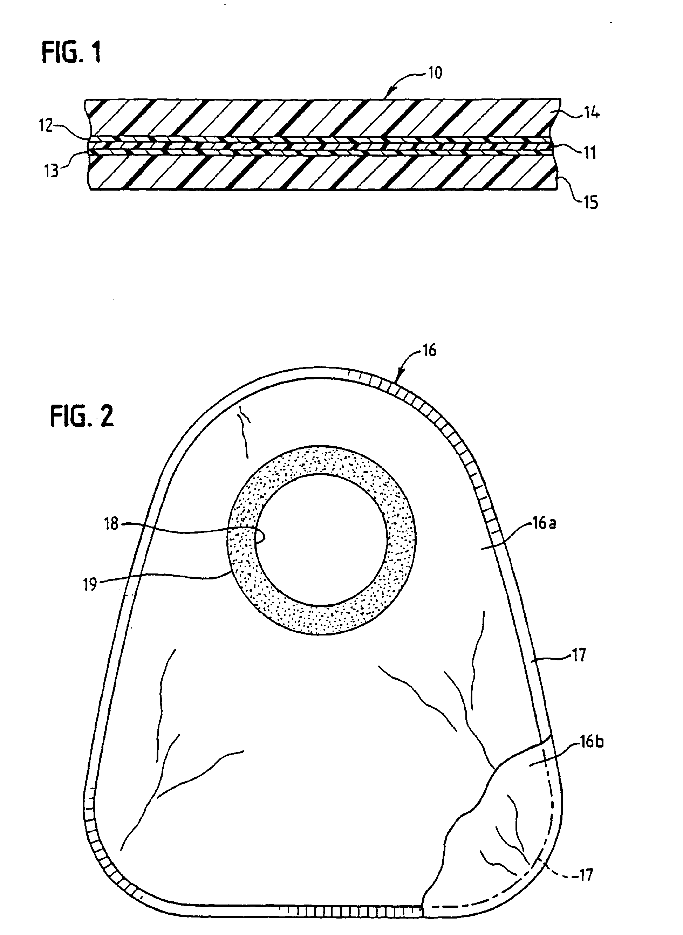

Figure 1 schematically illustrates a multilayer chlorine-free film 10 having an odor barrier layer 11 sandwiched between tie layers 12 and 13 and skin layers 14 and 15. The chlorine-free barrier layer 11 is essentially composed of a blend of amorphous polyamide (nylon) resin and an anhydride-modified olefinic polymer or copolymer. Unlike crystalline polyamides which are entirely aliphatic, amorphous polyamides have a partially aromatic structure and are typically produced by the condensation of an aliphatic diamine with an aromatic diacid, or combination of diacids, in molar amounts equivalent to the diamine used. While it is believed that any amorphous polyamide resin may be used, effective results have been obtained with a polyamide resin marketed as Selar PA3426 by Dupont Company. Selar PA3426 is understood to be substantially amorphous with a density of about 1.19 grams per cubic centimeter (g/cc). It has high melt strength and can be used under a broader range of processing conditions than conventional crystalline nylons. Selar PA3426 is produced by the condensation of.hexamethylenediamine, terephthalic acid, and isophthalic acid such that 65% to 80% of the polymer units are derived from hexamethylene isophthalamide. For further information, reference may be had to 52 Fed. Reg. 26,667 (1987).

The amorphous polyamide resin is the major constituent of the blend, comprising 70% to 90% by weight of that blend. The anhydride-modified olefinic polymer or copolymer comprises 10% to 30%, preferably 15% to 25% of the total weight of the barrier layer. The density of the anhydride modified olefinic polymer should be less than 0.89g/cc, preferably less than 0.87g/cc. Anhydride-modified olefinic polymers with densities higher than 0.89g/cc might still be effective as toughening agents but would not provide the reduction in film modulus and noise imparted by lower density polymers. The olefinic polymer or copolymers are functionalized by reactive processing with an unsaturated carboxylic anhydride. Although the mechanism of anhydride modification is not fully understood, it is believed that a grafting reaction occurs between the polymer and the unsaturated anhydride. While it is believed that other unsaturated carboxylic anhydrides may be used to provide the functional groups, maleic anhydride is considered particularly effective for that purpose. The level of maleic anhydride needed to functionalize the olefinic polymer is quite low, less than 2% by weight. The density of the olefinic polymer is essentially unaffected by anhydride modification at these low levels. One example of an anhydride-modified olefinic copolymer is Fusabond MN493D available from DuPont Company. Fusabond MN493D is an ethylene octene copolymer that is modified with 0.5% maleic anhydride and has a density of 0.869g/cc. While it is known to function as a toughening agent for crystalline nylon, Fusabond MN493D performs an unexpected function here in decreasing modulus and noise of amorphous nylon without destroying or significantly reducing the odor barrier properties of layer 11. Similar performance can be achieved with other anhydride-modified olefinic polymers having comparable low density, such as ethylene-propylene copolymers and terpolymers (EPM and EPDM). EPM and EPDM have a density in the 0.85 to 0.86g/cc range and are suitable for modification with maleic anhydride. Due to the immiscible nature of these blends, mixing of the nylon with the anhydride-modified olefinic polymer is best conducted in a separate step using a twin screw compounder extruder with either corotating or counterrotating screws. This allows an intimate dispersion of the olefinic phase into the nylon phase. The compounded blend is extruded and cut into pellets which can then be used for extrusion into film.

Skin layers 14 and 15 are formed of an ethylene-based polymer or copolymer with an alpha-olefin such as hexene or octene. A suitable resin is metellocene-catalyzed polyethylene with an octene comonomer such as the ethylene octene copolymer marketed under the designation Exact 8201 by Exxon Chemical.

Tie layers 12 and 13 must be capable of bonding to both the skin layers and the core barrier layer. Polyethylene, ethylene vinyl acetate copolymers (EVA), or ethylene methyl acrylate copolymers (EMA), modified with functional anhydride groups are believed particularly suitable. EVA-based anhydride-modified resins such as Bynel 3860 or Bynel 3861, or polyethylene-based anhydride-modified resins such as Bynel 41E557, all available from DuPont Company, have been found suitable. The ability of these anhydride-modified resins to act as adhesion promoters is believed to be due to an interfacial reaction between the anhydride groups in the tie layer and the amine groups of nylon in the barrier layer.

The total thickness of the multilayer film, assuming five layers are present, should fall within the general range of 0.0508 to 0.127 mm (2 to 5 mil) preferably 0.0762 to 0.1016 mm (3 to 4 mil). As to the barrier layer 11, its thickness should fall generally within the range of 0.00254 to 0.0254 mm (0.1 to 1.0 mil) with the lower limit being established by the capability of the extrusion process and the upper limit by the physical properties contributed by the barrier layer in achieving a multilayer film having low modulus and low noise characteristics. Preferably, the barrier layer thickness should fall within the range of 0.00508 to 0.01016 mm (0.2 to 0.4 mil) with 0.00762 mm (0.3 mil) being considered optimal when factors such as odor barrier properties, softness, quietness, and ease of extrusion are all considered together. By contrast, the skin layers 14 and 15 are each considerably thicker than the barrier layer sandwiched between them. For example, each skin layer may have a thickness within the general range of 0.0127 to 0.0635 mm (0.5 to 2.5 mil), preferably 0.0254 to 0.0508 mm (1 to 2 mil), which may be nearly one order of magnitude greater than the thickness of the odor barrier layer 11.

Figure 2 illustrates a typical ostomy pouch 16 having its walls 16a and 16b formed from the multilayer film of Figure 1. The films are arranged with their heat sealable skin layers facing each other and sealed together along the outer periphery of the pouch as indicated at 17. One wall of the pouch has a stoma-receiving opening 18 formed therein and an adhesive attachment ring 19 is located about that opening for adhesive attachment to the peristomal skin surfaces of a patient. The pouch as shown is of the type generally referred to as a one-piece appliance but, if desired, a mechanical coupling ring may be substituted for adhesive ring 19, with the pouch therefore becoming one component of a two-piece ostomy appliance, all as well known in the art.

In order that the invention may be more readily understood, reference is made to the following examples which are intended to be illustrative of the invention.

### Comparative Example 1

This example illustrates the properties of blends of amorphous nylon (Selar PA3426, DuPont Company) with anhydride-modified polyolefin (Fusabond MN493D, DuPont Company). The two resins were compounded and pelletized using a twin screw compounder extruder. The compounded resins were then coextruded using a Killion extruder into a A/B/A three-layer film structure, wherein A was polyethylene and B was the nylon blend. Because of the absence of tie layers, it was possible to strip the polyethylene skin layers A from the nylon blend layer B and to test the resulting monolayer B films against a monolayer film consisting of 100% amorphous nylon (Selar PA3426). Secant modulus at 2% elongation of the monolayer films was measured in the machine and transverse directions according to ASTM D882 at a strain rate of 0.254 cm/cm.min (0.1 in./in.min). Results are shown below in Table 1:

**Table 1**

| **Monolayer Selar PA3426** | **Film Composition Fusabond MN493D.** | **Secant Modulus psi** | |
|---|---|---|---|
| **%** | **%** | **MD** | **TD** |
| 100 | 0 | 323,500 | 312,600 |
| 85 | 15 | 235,600 | 215,000 |
| 75 | 25 | 190,000 | 120,000 |

The data in Table 1 shows the reduction in modulus resulting from the addition of Fusabond MN493D to Selar PA3426. The lower modulus values indicate that the compounded resins are considerably softer than the control resin with 100% amorphous nylon. The reduction in modulus allows the production of nylon-based multilayer films which are soft and quiet, as indicated in further examples below.

### Example 2

A five-layer film was produced in accordance with this invention by coextrusion casting, resulting in a film with a total thickness of 0.08382 mm (3.3 mil) and a barrier layer thickness of 0.008128 mm (0.32 mil). The film structure was A/B/C/B/A, where A was a polyethylene-based resin (Exact 8201, Exxon Chemical Co.) modified by the addition of 5% of a slip/antiblock concentrate (EXT4226TSE, A. Schulman Co.) and 3% of a low-density polyethylene (LD200.48, Exxon Chemical Co.). B are tie layers consisting of anhydride-modified ethylene vinyl acetate copolymer (Bynel 3601, DuPont Co.), and C is a blend of amorphous nylon (Selar PAJ426) with an anhydride-modified rubbery polyolefin (Fusabond MN493D) at 85% to 15% weight ratio.

The film was tested for quietness by forming a 10.16 cm (4 inch) by 10.16 cm (4 inch) sample into a cylinder and mounting it on a test fixture wherein one end of the cylinder was held fixed and the other was rotated around the cylinder axis at an angle of 15 degrees at 70 cycles per minute. Noise emissions produced by the film's flexing were analyzed with a sound level meter. For comparison, the same test was conducted on a commercial ostomy film with a chlorinated barrier. Results are shown below:

**Table 2**

| | **Sample** | **dBA** | **dB,8kHz** | **dB,16kHz** |
|---|---|---|---|---|
| | Film of Example 2 | 64 | 49 | 39 |
| | Control Film | 74 | 55 | 49 |

In this table, dBA is a weighted average that takes into account the human perception of noise over the entire frequency range, whereas dB values in the 8 and 16kHz octave bands are indicative of the noise in the higher frequency range and represent the crispness of the noise. The dBA and dB values therefore reveal that the film sample embodying the invention is considerably quieter than the control sample in which the core layer is based on PVDC.

### Example 3

A five-layer film was produced in accordance with this invention by coextrusion casting, resulting in a film with a total thickness of 0.08128 cm (3.2 mil) and a barrier layer thickness of 0.007112 cm (0.28 mil). The film construction was A/B/C/B/A, having the same composition as the film of Example 2 except that the tie layers B were polyethylene-based (Bynel 41E557, DuPont Co.). The film was tested for quietness as described in Example 2. Results are shown in the table below which includes a control sample of a commercial ostomy film having a chlorinated barrier layer of PVDC.

**Table 3**

| | **Sample** | **dBA** | **dB,8kHz** | **dB,16kHz** |
|---|---|---|---|---|
| | Film of Example 3 | 65 | 51 | 45 |
| | Control Film | 74 | 55 | 49 |

As in Example 2, the dBA and dB values at 8 and 16kHz reveal that the film sample of Example 3 is considerably quieter than the control sample in which the core layer is PVDC.

### Example 4

The films of Examples 2 and 3 were tested for odor transmission using British Standard 7127, Part 101, Appendix G: Method for Determining Odour Transmission of Colostomy and Ileostomy Bag Materials, British Standard Institution, London. Both films passed the test, indicating that the modification of the nylon barrier layer does not have a detrimental effect on odor barrier properties of the films.

In addition, a quantitative test of the barrier properties of the film of Example 2 was conducted using three model compounds for fecal odor: dimethyldisulfide, indole, and skatole. For comparison, the same test was conducted on a commercial ostomy film with a chlorinated (PVDC) barrier layer. Analysis of effluent gases was conducted by gas chromatography using a flame ionization detector. Table 4 shows breakthrough times and concentration of each component in the effluent stream after 60 hours.

**Table 4**

| Film | Breakthrough Times, min | | | Concentration at 60 hours | | |
|---|---|---|---|---|---|---|
| | Dimethyl disulfide min | Indole min | Skatole min | Dimethyl disulfide ppm | Indole ppb | Skatole ppb |
| Film of Example 2 | 2680 | 1880 | 2180 | 25 | 102 | 51 |
| Control Film | 722 | 1140 | 1610 | 137 | 292 | 91 |

Better barrier properties are expected for films that show longer breakthrough times and lower effluent concentration. The film of Example 2 is superior to the chlorinated control film in both respects, indicating superior performance as a barrier to fecal odorants.

## Claims

1. A multilayer chlorine-free film comprising an odor barrier layer of amorphous polyamide resin blended with 10% to 30% per total weight barrier layer of an anhydride-modified olefinic polymer or copolymer having a density of 0.89g/cc or lower, at least one heat-sealable skin layer of an ethylene polymer or copolymer, or blends thereof, and an adhesive tie layer of an anhydride-modified ethylene polymer or copolymer interposed between said skin layer and said barrier layer.

2. The film of Claim 1 in which said olefinic polymer or copolymer of said odor barrier layer is modified with 0.1% to 2% maleic anhydride.

3. The film of Claim 2 in which said olefinic polymer or copolymer of said barrier layer is an ethylene octene copolymer modified with 0.5% maleic anhydride.

4. The film of Claim 3 in which said amorphous polyamide resin is the condensation product of hexamethylenediamine, terephthalic acid, and isophthalic acid, and in which 65% to 80% of the polymer units are derived from hexamethylene isophthalamide.

5. The film of Claim 1 in which said anhydride-modified olefinic polymer or copolymer is present in the range of 15% to 25% per total weight barrier layer.

6. The film of Claims 1, 2 or 5 in which two of said skin layers and two of said adhesive tie layers are provided on opposite sides of said barrier layer.

7. The film of Claim 1 in which said barrier layer has a thickness within the range of 0.00254 to 0.0264 mm (0.1 mil to 1 mil) and the total thickness of said film is within the range of (2 mil to 5 mil) 0.0508 to 0.127 mm.

8. The film of Claim 7 in which said barrier layer has a thickness within the range of 0.00508 to 0.01016 mm (0.2 to 0.4 mil) and the total thickness of said film is (3 to 4 mil) 0.0762 to 0.1016 mm.

9. The film of Claim 6 in which said skin layers consist essentially of polyethylene or a copolymer of ethylene and an alpha olefin.

10. The film of Claim 6 in which said adhesive tie layers consist essentially of anhydride-modified polyethylene or anhydride-modified ethylene vinyl acetate copolymer.

11. An ostomy pouch having two side walls each comprising a multilayer chlorine-free film having an odor barrier layer according to claim 1 coextruded with at least one heat-sealable skin layer and with a tie layer interposed between each skin layer and barrier layer; said skin layers of said walls facing each other and being heat sealed along peripheral edge portions of said pouch; said barrier layer of each wall comprising amorphous polyamide resin blended with 10% to 30% per total weight barrier layer of an anhydride-modified olefinic polymer or copolymer having a density of 0.89g/cc or lower; said skin layers each comprising an ethylene polymer or copolymer or blend thereof; and said tie layers each comprising an anhydride-modified ethylene polymer or copolymer.

12. The pouch of Claim 11 in which said olefinic polymer or copolymer of said odor barrier layer is modified with 0.1% to 2% maleic anhydride.

13. The pouch of Claim 11 in which said olefinic polymer or copolymer of said barrier layer is an ethylene octene copolymer modified with 0.5% maleic anhydride.

14. The pouch of Claims 11 or 13 in which two of said skin layers and two of said tie layers are provided on opposite sides of each of said barrier layers.

15. The pouch of Claim 14 in which said skin layers consist essentially of polyethylene or a copolymer of ethylene and an alpha olefin.

16. The pouch of Claim 14 in which each of said adhesive tie layers consists essentially of anhydride-modified polyethylene or anhydride-modified ethylene vinyl acetate copolymer.

17. The pouch of Claim 14 in which said barrier layer of each film has a thickness within the range of 0.00508 to 0.01016 mm (0.2 mil to 0.4 mil and the total thickness of each film is within the range of (3 mil to 4 mil) 0.07662 to 0.1016 mm.

18. The pouch of Claim 11 in which said amorphous polyamide resin of each said film is the condensation product of hexamethylenediamine, terephthalic acid, and isophthalic acid, and in which 65% to 80% of the polymer units are derived from hexamethylene isophthalamide.

19. The pouch of Claim 11 in which said anhydride-modified olefinic polymer or copolymer is present in the range of 15% to 25% per total weight barrier layer.

## Patentansprüche

1. Mehriagiger chlorfreier Film, umfassend eine Geruchsbarriereschicht aus einem amorphen Polyamidharz, vermischt mit 10 bis 30%, bezogen auf das Gesamtgewicht der Barriereschicht, eines Anhydrid modifizierten olefinischen Polymers oder Copolymers mit einer Dichte von 0,89g/cm³ oder weniger, mindestens eine Heißkleberhautschicht aus einem Ethylenpolymer oder Copolymer, oder Mischungen davon, und eine adhesive Bindungsschicht aus einem Anhydrid modifizierten Ethylenpolymer oder Copolymer, vorgesehen zwischen besagter Hautschicht und besagter Barriereschicht.

2. Film nach Anspruch 1, wobei besagtes olefinisches Polymer oder Copolymer besagter Geruchsbarriereschicht mit 0,1 bis 2% Maleinsäureanhydrid modifiziert ist.

3. Film nach Anspruch 2, wobei besagtes olefinisches Polymer oder Copolymer besagter Barriereschicht ein Ethylen-Octen-Copolymer ist, modifiziert mit 0,5% Maleinsäureanhydrid.

4. Film nach Anspruch 3, wobei besagtes amorphes Polyamidharz das Kondensationsprodukt von Hexamethylendiamin, Terephthalsäure und Isophthalsäure ist, wobei 65 bis 80% der Polymereinheiten abgeleitet sind von Hexamethylenisophthalamid.

5. Film nach Anspruch 1, wobei besagtes Anhydrid modifiziertes olefinisches Polymer oder Copolymer in einer Menge von 15 bis 25% vorliegt, bezogen auf das Gesamtgewicht der Barriereschicht.

6. Film nach Anspruch 1, 2 oder 5, wobei zwei besagte Hautschichten und zwei besagte adhesive Bindungsschichten auf gegenüberliegenden Seiten besagter Barriereschicht vorgesehen sind.

7. Film nach Anspruch 1, wobei besagte Barriereschicht eine Dicke im Bereich von 0,00254 bis 0,0254 mm (0,1 mil bis 1 mil) aufweist und die Gesamtdicke besagten Films im Bereich von 0,0508 bis 0,127 mm (2 mil bis 5 mil) liegt.

8. Film nach Anspruch 7, wobei besagte Barriereschicht eine Dicke im Bereich von 0,00508 bis 0,01016 mm (0,2 mil bis 0,4 mil) aufweist und die Gesamtdicke besagten Filmes 0,0762 bis 0,1016 mm (3 mil bis 4 mil) beträgt.

9. Film nach Anspruch 6, wobei besagte Hautschichten im wesentlichen aus Ethylen oder einem Copolymer von Ethylen und einem alpha-Olefin bestehen.

10. Film nach Anspruch 6, wobei besagte adhesive Bindungsschichten im wesentlichen aus Anhydrid modifiziertem Polyethylen oder Anhydrid modifiziertem Ethylen-Vinylacetat-Copolymer bestehen.

11. Ostomiebeutel mit zwei Seitenwänden, jeweils umfassend einen mehrlagigen chlorfreien Film mit einer Geruchsbarriereschicht in Übereinstimmung mit Anspruch 1, coextrudiert mit mindestens einer Heißkleberhautschicht und mit einer Bindungsschicht, vorgesehen zwischen jeder Hautschicht und der Barriereschicht, wobei die Hautschichten besagter Wände einander gegenüberliegen und entlang der peripheralen Kantenteile besagten Beutels miteinander heißverklebt sind, wobei besagte Barriereschicht jeder Wand amorphes Polyamidharz umfasst, vermischt mit 10 bis 30%, bezogen auf das Gesamtgewicht der Barriereschicht, eines Anhydrid modifizierten olefinischen Polymers oder Copolymers mit einer Dichte von 0,89g/cm³ oder weniger, wobei besagte Hautschichten jeweils ein Ethylenpolymer oder Copolymer oder Mischungen daraus umfassen, und wobei besagte Bindungsschichten jeweils ein Anhydrid modifiziertes Ethylenpolymer oder Copolymer umfassen.

12. Beutel nach Anspruch 1, wobei besagtes olefinisches Polymer oder Copolymer besagter Geruchsbarriereschicht mit 0,1 bis 2% Maleinsäureanhydrid modifiziert ist.

13. Beutel nach Anspruch 11, wobei besagtes olefinisches Polymer oder Copolymer besagter Barriereschicht ein Ethylen-Octen-Copolymer, modifiziert mit 0,5% Maleinsäureanhydrid ist.

14. Beutel nach Anspruch 11 oder 13, wobei zwei besagte Hautschichten und zwei besagter Bindungsschichten auf gegenüberliegenden Seiten jeder der besagten Barriereschichten vorgesehen sind.

15. Beutel nach Anspruch 14, wobei besagte Hautschichten im wesentlichen aus Polyethylen oder einem Copolymer von Ethylen und einem alpha-Olefin bestehen.

16. Beutel nach Anspruch 14, wobei jede besagter adhesiven Bindungsschichten im wesentlichen aus Anhydrid modifiziertem Polyethylen oder Anhydrid modifiziertem Ethylen-Vinylacetat-Copolymer besteht.

17. Beutel nach Anspruch 14, wobei besagte Barriereschicht jedes Films eine Dicke im Bereich von 0,00508 bis 0,01016 mm (0,2 mil bis 0,4 mil) aufweist und die Gesamtdicke jedes Films im Bereich von 0,0762 bis 0,1016 mm (3 mil bis 4 mil) liegt.

18. Beutel nach Anspruch 11, wobei besagtes amorphes Polyamidharz jedes der besagten Filme das Kondensationsprodukt von Hexamethylendiamin, Terephthalsäure und Isophthalsäure ist, wobei 65 bis 80% der Polymereinheit abgeleitet sind von Hexamethylenisophthalamid.

19. Beutel nach Anspruch 11, wobei besagtes Anhydrid modifiziertes olefinisches Polymer oder Copolymer in einer Menge von 15 bis 25% vorliegt, bezogen auf das Gesamtgewicht der Barriereschicht.

## Revendications

1. Film multicouche exempt de chlore comprenant une couche de barrière aux odeurs d'une résine polyamide amorphe mélangée avec 10 % à 30 % du poids total de la couche de barrière d'un polymère ou copolymère oléfinique modifié par un anhydride ayant une masse volumique de 0,89 g/cm³ ou inférieure, au moins une couche de peau thermoscellable d'un polymère ou copolymère d'éthylène, ou de mélanges de ceux-ci, et une couche de lien adhésive d'un polymère ou copolymère d'éthylène modifié par un anhydride intercalée entre ladite couche de peau et ladite couche de barrière.

2. Film selon la revendication 1, dans lequel ledit polymère ou copolymère oléfinique de ladite couche de barrière aux odeurs est modifiée avec 0,1 % à 2 % d'anhydride maléique.

3. Film selon la revendication 2, dans lequel ledit polymère ou copolymère oléfinique de ladite couche de barrière est un copolymère éthylène-octène modifié avec 0,5 % d'anhydride maléique.

4. Film selon la revendication 3, dans lequel ladite résine polyamide amorphe est le produit de condensation de l'hexaméthylènediamine, l'acide téréphtalique et l'acide isophtalique, et dans lequel 65 % à 80 % des motifs du polymère sont dérivés de l'hexaméthylène isophtalamide.

5. Film selon la revendication 1, dans lequel ledit polymère ou copolymère oléfinique modifié par un anhydride est présent dans la gamme de 15 % à 25 % du poids total de la couche de barrière.

6. Film selon la revendication 1, 2 ou 5, dans lequel deux desdites couches de peau et deux desdites couches de lien adhésive sont disposées sur les côtés opposés de ladite couche de barrière.

7. Film selon la revendication 1, dans lequel ladite couche de barrière a une épaisseur dans la gamme de 0,00254 à 0,0254 mm (0,1 millième de pouce à 1 millième de pouce) et l'épaisseur totale dudit film est dans la gamme de 0,0508 à 0,127 mm (2 millièmes de pouce à 5 millièmes de pouce).

8. Film selon la revendication 7, dans lequel ladite couche de barrière a une épaisseur dans la gamme de 0,00508 à 0,01016 mm (0,2 à 0,4 millième de pouce) et l'épaisseur totale dudit film est de 0,0762 à 0,01016 (3 à 4 millièmes de pouce).

9. Film selon la revendication 6, dans lequel lesdites couches de peau consistent essentiellement en du polyéthylène ou un copolymère d'éthylène et d'une alpha oléfine.

10. Film selon la revendication 6, dans lequel lesdites couches de lien adhésives consistent essentiellement en un polyéthylène modifié par un anhydride ou un copolymère éthylène-acétate de vinyle modifié par un anhydride.

11. Poche stomique ayant deux parois latérales comprenant chacune un film multicouche exempt de chlore ayant une couche de barrière aux odeurs selon la revendication 1 co-extrudée avec au moins une couche de peau thermoscellable et avec une couche de lien intercalée entre chaque couche de peau et couche de barrière ; lesdites couches de peau desdites parois faisant face les unes aux autres et étant thermoscellées le long des parties de bord périphériques de ladite poche ; ladite couche de barrière de chaque paroi comprenant une résine polyamide amorphe mélangée avec 10 % à 30 % du poids total de la couche de barrière d'un polymère ou copolymère oléfinique modifié par un anhydride ayant une masse volumique de 0,89 g/cm³ ou inférieure ; lesdites couches de peau comprenant chacune un polymère ou copolymère d'éthylène ou mélange de ceux-ci : et lesdites couches de lien comprenant chacune un polymère ou copolymère d'éthylène modifié par un anhydride.

12. Poche selon la revendication 11, dans laquelle ledit polymère ou copolymère oléfinique de ladite couche de barrière aux odeurs est modifiée avec 0,1 % à 2 % d'anhydride maléique.

13. Poche selon la revendication 11, dans laquelle ledit polymère ou copolymère oléfinique de ladite couche de barrière est un copolymère d'éthylène-octène modifié avec 0,5 % d'anhydride maléique.

14. Poche selon la revendication 11 ou 13, dans laquelle deux desdites couches de peau et deux desdites couches de lien sont disposées sur des côtés opposés de chacune desdites couches de barrière.

15. Poche selon la revendication 14, dans laquelle lesdites couches de peau consistent essentiellement en du polyéthylène ou un copolymère d'éthylène et d'une alpha oléfine.

16. Poche selon la revendication 14, dans laquelle chacune desdites couches de lien adhésive consiste essentiellement en un copolymère de polyéthylène modifié par un anhydride ou éthylène-acétate de vinyle modifié par un anhydride.

17. Poche selon la revendication 14, dans laquelle ladite couche de barrière de chaque film a une épaisseur dans la gamme de 0,00508 à 0,01016 mm (0,2 millième de pouce à 0,4 millième de pouce) et l'épaisseur totale de chaque film est dans la gamme de 0,0762 à 0,1016 mm (3 millièmes de pouce à 4 millièmes de pouce).

18. Poche selon la revendication 11, dans laquelle ladite résine polyamide amorphe de chacun desdits films est le produit de condensation de l'hexaméthylènediamine, l'acide téréphtalique et l'acide isophtalique, et dans lequel 65 % à 80 % des motifs du polymère sont dérivés de l'hexaméthylène isophtalamide.

19. Poche selon la revendication 11, dans laquelle ledit polymère ou copolymère oléfinique modifié par un anhydride est présent dans la gamme de 15 % à 25 % du poids total de la couche de barrière.
